# EUROPEAN PATENT SPECIFICATION

(11) **EP 4 424 233 B1**
(45) Date of publication and mention of the grant of the patent: **23.04.2025**
(21) Application number: 23191467.2
(22) Date of filing: 15.08.2023
(51) Int. Cl.: A61M 16/00, A61M 16/14, A61B 5/083, A61M 11/00

(54) **ATOMIZED DRUG DELIVERY SYSTEM**
SYSTEM ZUR VERABREICHUNG ZERSTÄUBTER ARZNEIMITTEL
SYSTÈME D'ADMINISTRATION DE MÉDICAMENT ATOMISÉ

(30) Priority: 28.02.2023 CN 202310231740
(43) Date of publication of application: 04.09.2024
(73) Proprietor: Feellife Health Inc., Shenzhen, Guangdong 518000 (CN)
(72) Inventor: HUA, Jian, Shenzhen, 518000 (CN); LU, Jialin, Shenzhen, 518000 (CN)
(74) Representative: Purdylucey Intellectual Property

(56) References cited:
- WO-A1-2009/042187
- WO-A1-2022/231134
- CA-A1- 3 057 400
- CN-U- 213 609 045
- US-A- 4 787 655
- US-A- 5 964 219
- US-A1- 2012 136 269
- US-A1- 2022 293 262

## Description

### TECHNICAL FIELD

The present application relates to the field of medical devices, and particularly to an atomized drug delivery system and a control method thereof.

### BACKGROUND

At present, ventilators have been widely used in the medical field. The ventilators are medical devices that can replace spontaneous ventilation function, which are mostly used in patients with respiratory failure, anesthesia respiratory management during surgery, respiratory supportive treatment and emergency resuscitation, and can prevent and treat respiratory failure, reduce complications and save patients' lives. Generally, the ventilator is used in conjunction with an atomizer for atomizing a drug into tiny particles, which are then inhaled by the patient through respiration, reaching the respiratory tract and lungs, thereby achieving painless, rapid, and effective treatment.

WO2022/231134A1 discloses a drug aerosol supply apparatus for a respirator comprises: an air purifier discharging first air; an inhaled air sensor for generating a sensing signal by sensing the first air; a heating and humidifying apparatus discharging second air which is formed by adjusting the temperature and the humidity of the first air; a drug fine particle generator operating only when the sensing signal is input, to spray a drug in a fine particle state to the second air; a Y-tube having one end connected to a T-tube and another end connected to a mask or a mouthpiece; and a return tube having one end connected to the Y-tube and another end connected to the air purifier to allow exhaled air to flow.

US2022/293262A1 discloses a context sensitive guidance (CSG) system for providing clinical interventions to a patient in an emergency medical event includes a CSG engine, patient interface devices configured to generate signals indicative of patient physiologic data, and a display device configured to provide a CSG user interface and medical device(s) configured to couple to the patient interface devices, the CSG engine and the display device, receive the signals, generate the physiologic data from the signals, and send the physiologic data to the CSG engine and the display device, wherein the CSG engine is configured to receive and evaluate the physiologic data, identify a clinical intervention, and send an output based on the clinical intervention to the medical device(s) configured to perform at least one operation in response to the sent output.

US2012/136269A1 discloses a liquid separation apparatus for removing a liquid from a respiratory gas is provided. The liquid separation apparatus comprises a housing that comprises an input, wherein a sample withdrawn from the respiratory gas is delivered through the input; a first output wherein at least part of the sample of the respiratory gas is discharged through the first output; and a first filter configured to remove the liquid from the sample of respiratory gas flowing through the input and the first output of the housing. The first filter comprises a hydrophobic property and an oleophobic property to remove the liquid when a surface tension of the liquid is lower than a surface tension of bodily fluids.

US4787655A discloses an airtight connection for breathing circuits. The invention comprises a swivel adapter for a ventilator breathing system serving an incapacitated patient. The swivel adapter utilizes a pair of corrugated hoses which mate with a pair of arms which are permitted to swivel on the body of the adapter. A ribbed collar is disposed on the body of the adapter and receives an endotracheal tube inserted within the trachea of the patient. A locking means secures the branches of the body to the swivelable arms of the adapter, and a circumferential sealing means, in the form of an 'o'-ring is disposed between the branch and its receiving orifice in its respective arm, to permit secure swiveling thereof, while preventing leakage of any gases therefrom.

US5964219A discloses a method and an arrangement for processing respiratory gas of an intubated patient, wherein the level of humidity of the inhalation gas is increased, if necessary, before the gas is supplied to the patient. To provide accurate and correctly timed humidification, the patient's respiratory volume is measured, the amount of water discharged from the patient is determined on the basis of the measured respiratory volume, and a desired amount of water, dependent on the amount of water discharged from the patient, is supplied to the patient's respiratory tract during the next respiratory phase(s).

CN213609045U discloses a multi-functional flexible and portable atomization device, wherein it includes a host, a carrying frame mounted on the host for fixing the host, a Y-type multi-functional data cable electrically connected to the host, and an atomizer and a connector respectively arranged on the bifurcated end of the Y-type data cable.

In the related art, the atomizer is generally set to the same respiratory rate as the ventilator connected therewith to perform an atomizing operation. However, this operation is very prone to a non-synchronous phenomenon of atomization and respiration, which is very dangerous for patients and difficult to realize accurate drug delivery, and a related atomized drug delivery system cannot detect a parameter of exhaled gas from lungs.

### SUMMARY

The invention relates to an atomized drug delivery system according to the claims.

In some embodiments, the system further comprises a three-way pipe, the three-way pipe is provided with a first interface, a second interface and a third interface, the first interface is connected with the first ventilation pipeline, the second interface is connected with the second ventilation pipeline, and the third interface is connected with the face mask.

In some embodiments, the system further comprises a filter, one end of the filter is connected with the ventilator, the other end of the filter is connected with the first ventilation pipeline, and the filter is configured for filtering bacteria and impurities.

In some embodiments, the system further comprises a humidifier, one end of the humidifier is connected with the filter, the other end of the humidifier is connected with the first ventilation pipeline, the humidifier comprises a humidity assembly and a heating assembly, the humidity assembly is configured for adjusting humidity of exhaled gas of the ventilator, and the heating assembly is configured for adjusting temperature of the gas.

In some embodiments, the system further comprises an upper computer, the upper computer is connected with the ventilator, the atomizer and the spirometer, the upper computer comprises a test module and a display module, the test module is configured for sending a test instruction to the ventilator, the atomizer and/or the spirometer, so that the ventilator, the atomizer and/or the spirometer is capable of generating reply information according to the test instruction, and the display module is configured for displaying display information and graph obtained by processing the reply information.

Also disclosed is a control method of the atomized drug delivery system described, wherein the control method comprises: acquiring the respiratory signal detected by the respiratory sensor located on the first ventilation pipeline; determining the respiratory state through the respiratory signal; when the respiratory state is the inspiratory state, adjusting the frequency of the atomizing apparatus in the atomizer through the host machine of the atomizer, to control the atomizing apparatus to atomize the stored drug and deliver the atomized drug to the first ventilation pipeline; when the respiratory state is the expiratory state, adjusting the frequency of the atomizing apparatus through the host machine, to control the atomizing apparatus to stop atomizing the drug; and acquiring the exhaled gas through the spirometer located on the second ventilation pipeline, and calculating the vital capacity parameter according to the gas.

In some embodiments, the respiratory signal comprises a signal direction value, and the determining the respiratory state through the respiratory signal comprises: when the signal direction value indicates a first direction, determining that the respiratory state is the expiratory state; and when the signal direction value indicates a second direction, determining that the respiratory state is the inspiratory state.

In some embodiments, the respiratory signal comprises a signal magnitude, a respiratory threshold is set for the system, and the control method further comprises: when the signal magnitude is greater than the respiratory threshold, adjusting the frequency of the atomizing apparatus through the host machine, to control the atomizing apparatus to reduce a drug atomization amount; and when the signal magnitude is smaller than the respiratory threshold, adjusting the frequency of the atomizing apparatus through the host machine, to control the atomizing apparatus to increase the drug atomization amount.

In some embodiments, the calculating the vital capacity parameter according to the gas, comprises: sampling the gas at a high speed to obtain sampling data, and analyzing the sampling data to obtain a forced vital capacity and a peak flow velocity; collecting pressure information of the gas, obtaining a pressure analog signal value of the gas according to the pressure information, and calculating a gas capacity of the gas according to the pressure analog signal value; and taking at least one of the forced vital capacity, the peak flow velocity or the gas capacity as the vital capacity parameter.

In some embodiments, the system further comprises the upper computer, and the upper computer is connected with the ventilator, the atomizer and the spirometer; and the control method further comprises: sending the test instruction to the ventilator, the atomizer and/or the spirometer through the test module of the upper computer, so that the ventilator, the atomizer and/or the spirometer generates the reply information according to the test instruction; and sending the reply information to the upper computer, processing the reply information, and displaying the display information and graph obtained by processing through the display module of the upper computer.

The embodiments of the present application provides the atomized drug delivery system and the control method thereof. The respiratory state is determined by acquiring the respiratory signal detected by the respiratory sensor located on the first ventilation pipeline, then the frequency of the atomizing apparatus in the atomizer is adjusted according to the respiratory state, to control the atomizing apparatus to atomize or stop atomizing the stored drug, the exhaled gas is acquired by the spirometer located on the second ventilation pipeline, and the vital capacity parameter is calculated according to the exhaled gas.

According to the embodiments of the present application, the respiratory state can be detected by the respiratory sensor, the atomizing frequency of the atomizer is adjusted according to this state, and the vital capacity parameter is calculated according to the exhaled gas during atomized drug delivery, to detect a lung function of a patient under the atomized drug delivery system, so that a parameter related to exhaled gas from lungs can be detected in real time while realizing accurate drug delivery under the cooperation between the atomizer and the ventilator.

### BRIEF DESCRIPTION OF THE DRAWINGS

FIG. 1 is a schematic diagram of an atomized drug delivery system according to an embodiment of the present application;
FIG. 2 is an optional flow chart of a control method of the atomized drug delivery system according to an embodiment of the present application;
FIG. 3 is a flow chart of step S1002 in FIG. 2;
FIG. 4 is another optional flow chart of the control method of the atomized drug delivery system according to an embodiment of the present application;
FIG. 5 is a flow chart of step S1005 in FIG. 2; and
FIG. 6 is yet another optional flow chart of the control method of the atomized drug delivery system according to an embodiment of the present application.

### Reference numerals:

100 refers to face mask, 101 refers to first ventilation pipeline, 102 refers to second ventilation pipeline, 110 refers to ventilator, 120 refers to respiratory sensor, 130 refers to atomizer, 131 refers to host machine, 132 refers to atomizing apparatus, 140 refers to spirometer, 150 refers to three-way pipe, 160 refers to filter, 170 refers to humidifier, and 180 refers to upper computer.

### DETAILED DESCRIPTION

To make the objects, the technical solutions, and the advantages of the present application clearer, the present application is further described in detail hereinafter with reference to the drawings and embodiments. It should be understood that the specific embodiments described herein are only used for explaining the present application and are not intended to limit the present application.

It should be noted that although the functional module division is performed in the schematic diagram of the apparatus and the logical sequence is shown in the flow chart, in some cases, it is possible to use different module divisions than those in the apparatus or to execute the steps in a different sequence than shown or described in the flowchart.

Unless otherwise defined, all technical and scientific terms used herein have the same meanings as those commonly understood by those skilled in the art. The terms used herein are only for the purpose of describing the embodiments of the present application, and are not intended to limit the present application.

Atomized drug delivery is a common medical means, and refers to dispersing a drug into tiny droplets or particles by an atomizing apparatus, to make the tiny droplets or particles suspend in gas and enter respiratory tract and lungs, thus achieving the purpose of humidifying an air passage and relieving respiratory tract inflammation.

The atomized drug delivery can well relieve respiratory tract infection, atomized drug can directly reach throat, trachea, bronchi and even lungs, and it is more beneficial for coming into play and reducing side effects caused by drug delivery due to a higher local drug concentration.

Under normal circumstances, an atomizer may be used in conjunction with a ventilator, but in the related art, the atomizer is generally set to the same respiratory rate as the ventilator to perform an atomizing operation, which easily leads to a non-synchronous phenomenon of atomization and respiration, thus being very dangerous for a patient. In addition, the related atomized drug delivery system cannot detect a parameter related to exhaled gas from lungs of the patient.

On this basis, embodiments of the present application provide an atomized drug delivery system and a control method thereof, through which a parameter of exhaled gas from lungs can be detected while improving the accuracy of atomized drug delivery.

The atomized drug delivery system and the control method thereof in the embodiments of the present application may be illustrated by the following embodiments.

It should be noted that, in various embodiments of the present application, when related processing needs to be performed based on user's information, user's behavior data, user's historical data, user's position information and other data related to user's identity or characteristics, user's permission or consent will be obtained first. For example, the user's permission or consent will be obtained first when an access request for user's stored data and user's cache data is acquired. Moreover, the collection, use and processing of these data will comply with the relevant laws, regulations and standards of relevant countries and regions. In addition, when the embodiments of the present application need to acquire user's sensitive personal information, user's separate permission or consent will be obtained by a pop-up window, jumping to a confirmation page, or other modes, and after the user's separate permission or consent is explicitly obtained, necessary user-related data for normal operation of the embodiments of the present application will be acquired.

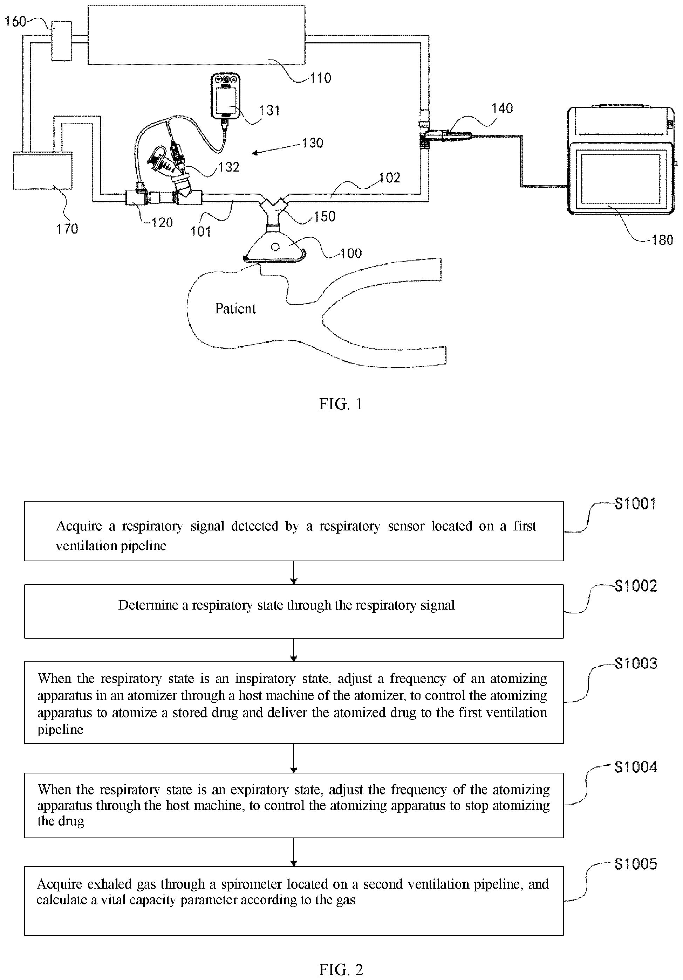

The embodiments of the present application is further described hereinafter with reference to FIG. 1 to FIG. 6, wherein reference numerals are all mentioned in FIG. 1 to FIG. 6.

The atomized drug delivery system and the control method thereof according to embodiments of the present application are described in detail hereinafter with reference to the following embodiments, and the atomized drug delivery system in the embodiments of the present application will be described first.

With reference to FIG. 1, FIG. 1 is a schematic diagram of an atomized drug delivery system according to an embodiment of the present application, and the system comprises:
a face mask, wherein the face mask is connected with a first ventilation pipeline and a second ventilation pipeline respectively, the first ventilation pipeline is configured for being connected with a ventilator, a respiratory sensor is arranged in the first ventilation pipeline, and the respiratory sensor is configured for detecting a respiratory signal;
an atomizer, wherein the atomizer is provided with a host machine and an atomizing apparatus, the host machine is connected with the atomizing apparatus and the respiratory sensor, the host machine is configured for receiving the respiratory signal detected by the respiratory sensor, and the atomizing apparatus is further connected with the first ventilation pipeline; wherein, the host machine is configured for determining a respiratory state through the respiratory signal, when the respiratory state is an inspiratory state, the host machine is configured for adjusting a frequency of the atomizing apparatus, to control the atomizing apparatus to atomize a stored drug and deliver the atomized drug to the first ventilation pipeline, and when the respiratory state is an expiratory state, the host machine is configured for adjusting the frequency of the atomizing apparatus through the host machine, to control the atomizing apparatus to stop atomizing the drug; and
a spirometer, wherein the spirometer is connected with the second ventilation pipeline, and the spirometer is configured for acquiring exhaled gas through the second ventilation pipeline and calculating a vital capacity parameter according to the gas.

In some embodiments, as shown in FIG. 1, a patient may be connected with the atomized drug delivery system through the face mask 100, the face mask 100 is connected with the ventilator 110 and the atomizer 130 through the first ventilation pipeline 101, and when the atomizer 130 performs atomized drug delivery, the atomized drug may reach the face mask 100 along with exhaled gas of the ventilator 110 through the first ventilation pipeline 101, so that the patient can inhale the drug through the face mask 100.

In some embodiments, the respiratory state refers to the expiratory state and the inspiratory state of the patient. It can be understood that, when the patient is in the inspiratory state, the ventilator is in the expiratory state, and at the moment, the host machine of the atomizer controls the atomizing apparatus to atomize the drug. When the patient is in the expiratory state, the ventilator is in the inspiratory state, and at the moment, the host machine of the atomizer controls the atomizing apparatus to stop atomizing the drug.

In some embodiments, the drug to be atomized may be placed in a drug reservoir, and there are many types of drug reservoirs, such as being box-shaped, bottle-shaped, or bag-shaped. The drug reservoir specifically needs to be selected according to drug characteristics of different drugs.

In some embodiments, the respiratory sensor 120 may be a gas flow sensor, and the gas flow sensor is a sensor capable of displaying instantaneous flow rate and direction. In the embodiment of the present application, the gas flow sensor may judge the respiratory state according to a direction of gas. For example, when the gas flow sensor detects that the gas of the ventilator 110 flows from the ventilator 110 towards the atomizer 130, it is indicated that the ventilator 110 is in the expiratory state and the patient is in the inspiratory state, and at the moment, the atomizer 130 atomizes the drug. When the gas flow sensor detects that the gas of the ventilator 110 flows from the atomizer 130 towards the ventilator 110, it is indicated that the ventilator 110 is in the inspiratory state and the patient is in the expiratory state, and at the moment, the atomizer 130 stops atomizing the drug.

In some embodiments, the respiratory sensor 120 may be a pressure sensor, and the gas pressure sensor is a sensor capable of measuring a pressure of gas. In the embodiment of the present application, the gas pressure sensor may judge the respiratory state according to positive or negative value of the pressure of gas. For example, when the gas pressure sensor reads that a pressure value of gas in the system is positive, it is indicated that there is a positive pressure in the system, at the moment, the ventilator 110 is in the expiratory state and the patient is in the inspiratory state, and the atomizer 130 atomizes the drug. When the gas pressure sensor reads that the pressure value of the gas in the system is negative, it is indicated that there is a negative pressure in the system, at the moment, the ventilator 110 is in the inspiratory state and the patient is in the expiratory state, and the atomizer 130 stops atomizing operation.

That is, the atomizing state of the atomizer 130 is relevant to the respiratory state detected by the respiratory sensor 120, and the atomizer 130 can perform the atomizing operation according to the respiratory state, thus realizing frequency synchronization between the atomizer 130 and the ventilator 110.

It should be noted that the respiratory sensor 120 may also be other sensors capable of judging the respiratory state, and the above embodiments are only preferred embodiments of the present application, which are not limited herein.

In some embodiments, the spirometer 140 is connected with the face mask 100 through the second ventilation pipeline 102. When the patient exhales, the exhaled gas can flow through the spirometer 140 through the second ventilation pipeline 102. At the moment, the spirometer 140 detects a parameter related to the exhaled gas of the patient, to show the effect after delivery with atomized drug.

In some optional embodiments, the atomized drug delivery system further comprises a three-way pipe, the three-way pipe is provided with a first interface, a second interface and a third interface, the first interface is connected with the first ventilation pipeline, the second interface is connected with the second ventilation pipeline, and the third interface is connected with the face mask.

In some embodiments, the three-way pipe 150 is mainly configured for changing a flow direction of gas. On one hand, the three-way pipe 150 enables the atomized drug to be delivered to the patient through the third interface connected with the face mask 100, and on the other hand, the exhaled gas can be delivered to the spirometer 140 through the second interface, to detect a parameter related to lung function.

In some optional embodiments, the atomized drug delivery system further comprises a filter, one end of the filter is connected with the ventilator, the other end of the filter is connected with the first ventilation pipeline, and the filter is configured for filtering bacteria and impurities.

In some embodiments, because the patient's respiratory tract is fragile, bacteria or fine impurities contained in the exhaled gas of the ventilator 110 may easily cause cross-infection between the patients, leading to the aggravation of disease. In the embodiment of the present application, a gas exhaling end of the ventilator 110 is connected with the filter 160 to filter pollutants such as bacteria and impurities in the gas.

It should be noted that the filter 160 may be disposable or reusable, which is not specifically limited in the present application.

In some optional embodiments, the atomized drug delivery system further comprises a humidifier, one end of the humidifier is connected with the filter, the other end of the humidifier is connected with the first ventilation pipeline, the humidifier comprises a humidity assembly and a heating assembly, the humidity assembly is configured for adjusting humidity of the exhaled gas of the ventilator, and the heating assembly is configured for adjusting temperature of the gas.

In some embodiments, the humidifier 170 comprises the humidity assembly, the humidity assembly can evaporate pure water added into the humidifier 170, to change liquid water into steam water, and increase the humidity of the exhaled gas of the ventilator 110, thus reducing irritation of dry gas to the patient's nasal cavity and respiratory tract.

In some embodiments, the humidifier 170 comprises the heating assembly, the heating assembly can heat the evaporated gas, so that temperature of the heated gas is maintained in a constant range for the comfort of the patient, thus reducing irritation of low-temperature gas to the patient's nasal cavity and respiratory tract.

In some optional embodiments, the atomized drug delivery system further comprises an upper computer, the upper computer is connected with the ventilator, the atomizer and the spirometer, the upper computer comprises a test module and a display module, the test module is configured for sending a test instruction to the ventilator, the atomizer or the spirometer, so that the ventilator, the atomizer or the spirometer is capable of generating reply information according to the test instruction, and the display module is configured for displaying display information and graph obtained by processing the reply information.

In some embodiments, the upper computer 180 is connected with the ventilator 110, the atomizer 130 and the spirometer 140, and the upper computer 180 is configured for sending the test instruction to the ventilator 110, the atomizer 130 and/or the spirometer 140 and displaying related test results. In addition, the upper computer 180 may also monitor operation conditions of the ventilator 110, the atomizer 130 and the spirometer 140.

Further, the test module of the upper computer 180 can send the test instruction to the ventilator 110, and the test instruction comprises a respiratory rate parameter and a respiratory intensity parameter related to operation of the ventilator 110. The ventilator 110 responds to the test instruction sent by the upper computer 180 and generates the reply information of the ventilator 110, and the reply information of the ventilator 110 comprises a parameter value for actual operation of the ventilator 110 under the test instruction. The reply information of the ventilator 110 is transmitted to the upper computer 180 through a serial port, and the upper computer 180 can generate the display information and graph after processing the reply information of the ventilator 110 and display the display information and graph through the display module.

Further, the test module of the upper computer 180 can send the test instruction to the atomizer 130, and the test instruction comprises an atomizing intensity parameter related to operation of the atomizer 130. The atomizer 130 responds to the test instruction sent by the upper computer 180 and generates the reply information of the atomizer 130, and the reply information of the atomizer 130 comprises a parameter value for actual operation of the atomizer 130 under the test instruction. The reply information of the atomizer 130 is transmitted to the upper computer 180 through a serial port, and the upper computer 180 can generate the display information and graph after processing the reply information of the atomizer 130 and display the display information and graph through the display module.

Further, the test module of the upper computer 180 can send the test instruction to the spirometer 140, and the test instruction comprises a lung function test parameter related to operation of the spirometer 140. The spirometer 140 can respond to the test instruction sent by the upper computer 180 and generates the reply information of the spirometer 140, and the reply information of the spirometer 140 comprises a parameter value for actual operation of the spirometer 140 under the test instruction. The reply information of the spirometer 140 is transmitted to the upper computer 180 through a serial port, and the upper computer 180 can generate the display information and graph after processing the reply information of the spirometer 140 and display the display information and graph through the display module.

Further, the upper computer 180 may monitor the ventilator 110, the atomizer 130 and the spirometer 140 in real time. If any abnormality occurs with the ventilator 110, the atomizer 130, or spirometer 140, such as data anomalies or excessive machine temperature, the host computer 180 can issue an alert.

With reference to FIG. 2, FIG. 2 is an optional flow chart of a control method of the atomized drug delivery system according to an embodiment of the present application, and the method in FIG. 2 comprises, but is not limited to step S1001 to step S1005.

In step S1001, the respiratory signal detected by the respiratory sensor located on the first ventilation pipeline is acquired.

In step S1002, the respiratory state is determined through the respiratory signal.

In some embodiments, the control method of the atomized drug delivery system may be applied to the atomized drug delivery system.

In some embodiments, the atomized drug delivery system does not comprise the ventilator 110, and the ventilators 110 with different functions may be externally connected to the atomized drug delivery system according to specific conditions of different patients, to perform targeted atomized drug delivery according to the conditions of different patients.

In some embodiments, the atomized drug delivery system comprises the ventilator 110, and the ventilator 110 is matched with the atomizer 130 in the atomized drug delivery system, and has a specific adjustable respiratory rate range. When a patient uses the atomized drug delivery system, the ventilator 110 cannot be replaced, so that unification between the ventilator 110 and the atomizer 130 is maintained, and when the atomized drug delivery system delivers a drug to the patient, an optimal drug delivery time will not be missed due to mismatching between the atomizer 130 and the newly connected ventilator 110.

In step S1003, when the respiratory state is the inspiratory state, the frequency of the atomizing apparatus in the atomizer is adjusted through the host machine of the atomizer, to control the atomizing apparatus to atomize the stored drug and deliver the atomized drug to the first ventilation pipeline.

In step S1004, when the respiratory state is the expiratory state, the frequency of the atomizing apparatus is adjusted through the host machine, to control the atomizing apparatus to stop atomizing the drug.

In some embodiments, the host machine 131 of the atomizer 130 can control an atomizing frequency of the atomizing apparatus 132 according to the respiratory state detected by the respiratory sensor 120. For example, when the respiratory sensor 120 detects that the respiratory state of the patient is the inspiratory state, the host machine 131 controls the atomizing apparatus 132 to atomize the stored drug, and the atomizing frequency may be adjusted. For example, when the atomized drug delivery system is just started, a relatively high atomizing frequency may be set, and after a gas flow in the system is stable, the atomizing frequency of the atomizer 130 may be reduced. When the respiratory sensor 120 detects that the respiratory state of the patient is the expiratory state, the host machine 131 controls the atomizing apparatus 132 to stop atomizing the stored drug. The atomizer 130 detects the respiratory state through the respiratory sensor 120, and then adjusts the atomizing frequency according to the respiratory state. When the ventilator 110 is in the inspiratory state, the patient is in the expiratory state, and the atomizer 130 stops atomizing the drug. When the ventilator 110 is in the expiratory state, the patient is in the inspiratory state, and the atomizer 130 atomizes the drug, so that the patient can inhale the atomized drug at the moment, thus achieving the purpose of accurate drug delivery.

In step S1005, the exhaled gas is acquired through the spirometer located on the second ventilation pipeline, and the vital capacity parameter is calculated according to the gas.

In some embodiments, the exhaled gas of the patient can reach the spirometer 140 through the second ventilation pipeline 102, and the spirometer 140 can calculate an activity parameter related to lungs according to the exhaled gas of the patient. The vital capacity parameter is used for indicating various parameter values of lung function of the current patient who is being delivered with atomized drug by the atomized drug delivery system, and is compared with a normal range to achieve the purpose of real-time detection of the lung function after delivery with atomized drug.

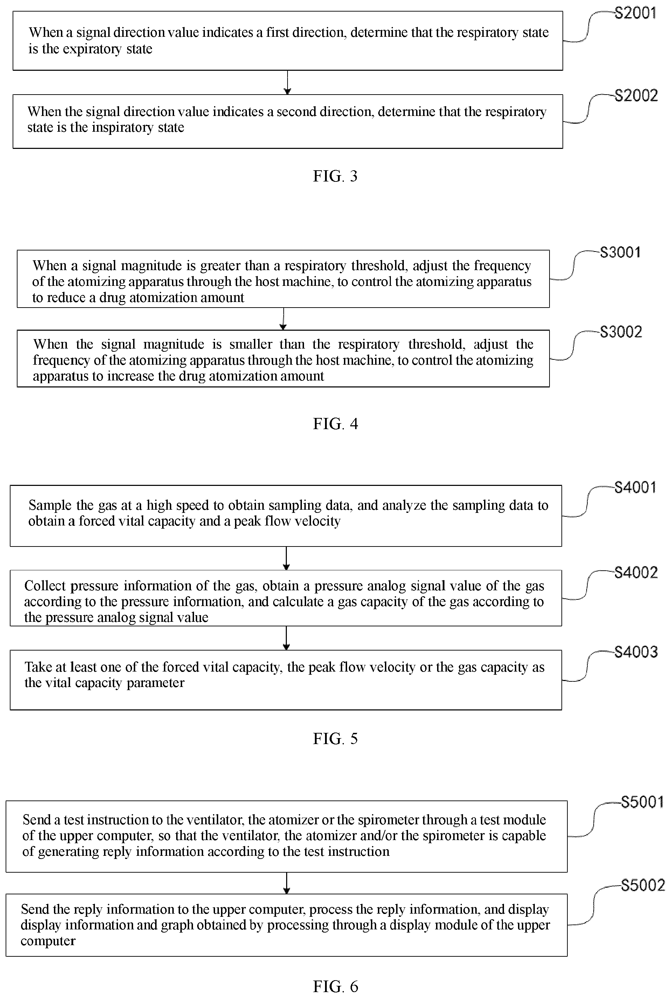

With reference to FIG. 3, FIG. 3 is a flow chart of the step S1002 in FIG. 2, and in some embodiments, the step S1002 may further comprise step S2001 to step S2002.

In step S2001, when a signal direction value indicates a first direction, it is determined that the respiratory state is the expiratory state.

In step S2002, when the signal direction value indicates a second direction, it is determined that the respiratory state is the inspiratory state.

In some embodiments, the first direction is set to be from the ventilator 110 to the atomizer 130, and the second direction is set to be from the atomizer 130 to the ventilator 110.

Alternatively, the signal direction value is divided into a positive value and a negative value. The signal direction value indicates the first direction when the signal direction value is positive, and the respiratory state of the ventilator 110 is the expiratory state, and the respiratory state of the patient is the inspiratory state. The signal direction value indicates the second direction when the signal direction value is negative, and the respiratory state of the ventilator 110 is the inspiratory state, and the respiratory state of the patient is the expiratory state.

With reference to FIG. 4, FIG. 4 is another optional flow chart of the control method of the atomized drug delivery system according to an embodiment of the present application. In some embodiments, the respiratory signal comprises a signal magnitude, and a respiratory threshold is set for the system. The method in FIG. 4 comprises, but is not limited to step S3001 to step S3002.

In step S3001, when the signal magnitude is greater than the respiratory threshold, the frequency of the atomizing apparatus is adjusted through the host machine, to control the atomizing apparatus to reduce a drug atomization amount.

In step S3002, when the signal magnitude is smaller than the respiratory threshold, the frequency of the atomizing apparatus is adjusted through the host machine, to control the atomizing apparatus to increase the drug atomization amount.

In some embodiments, the respiratory threshold is set for the system. Meanwhile, the respiratory threshold is adjustable, and the respiratory threshold is used for indicating a respiratory weakness degree of the patient. The signal magnitude may be a pressure value of one respiration. When the pressure value is greater than the preset respiratory threshold of the system, it is indicated that the patient has a strong spontaneous respiratory ability, and the drug atomization amount of the atomizing apparatus 132 may be reduced. When the pressure value is smaller than the preset respiratory threshold of the system, it is indicated that the patient has a weak spontaneous respiratory ability, and the drug atomization amount of the atomizing apparatus 132 may be increased.

Alternatively, the signal magnitude may be a respiratory rate over a period of time. When the respiratory sensor 120 detects that the respiratory rate of the patient is higher than the preset respiratory threshold of the system, it is indicated that the patient may not adapt to the atomized drug, and the drug atomization amount of the atomizing apparatus 132 may be reduced, and meanwhile, the upper computer 180 connected with the atomizer 130 gives an alert prompt. When the respiratory sensor 120 detects that the respiratory rate of the patient is lower than the preset respiratory threshold of the system, it is indicated that the patient respires weakly, and at the moment, the drug atomization amount of the atomizing apparatus 132 may be increased, to increase a drug amount inhaled by the patient each time.

With reference to FIG. 5, FIG. 5 is a flow chart of the step S1005 in FIG. 2, and in some embodiments, the step S1005 may further comprise step S4001 to step S4003.

In step S4001, the gas is sampled at a high speed to obtain sampling data, and the sampling data are analyzed to obtain a forced vital capacity and a peak flow velocity.

In step S4002, pressure information of the gas is collected, a pressure analog signal value of the gas is obtained according to the pressure information, and a gas capacity of the gas is calculated according to the pressure analog signal value.

In step S4003, at least one of the forced vital capacity, the peak flow velocity or the gas capacity is taken as the vital capacity parameter.

In some embodiments, the spirometer 140 can sample the exhaled gas of the patient at a high speed to obtain the sampling data, and analyze the sampling data to obtain the forced vital capacity, the peak flow velocity and other vital capacity parameters, wherein the forced vital capacity refers to a maximum amount of gas that can be exhaled as soon as possible after trying to inspire gas as much as possible, which is a primary indicator to judge a lung expansion ability; and the peak flow velocity refers to a maximum instantaneous flow velocity during measuring the forced vital capacity, which mainly reflects a strength of respiratory muscles and whether an air passage is blocked or not. The spirometer 140 obtains various vital capacity parameters from the exhaled gas of the patient, which realizes real-time detection of the lung function of the patient subjected to atomized drug delivery when the atomized drug delivery system performs the atomized drug delivery to the patient, to feed back an atomized drug delivery effect.

In some embodiments, a pressure sensor is arranged in the spirometer 140. When the pressure sensor detects that the patient is in the expiratory state, the pressure sensor can collect pressure information of the exhaled gas of the patient, convert the pressure information into a corresponding pressure analog signal value, and finally calculate a gas capacity of the exhaled gas of the patient, which is used for evaluating whether the respiration of the patient is normal or not.

In some embodiments, the forced vital capacity, the peak flow velocity and the gas capacity may all be used as lung activity parameters in lung function detection of the patient, and one of the parameters may be selected as the most important reference parameter according to actual needs, or the lung activity parameters may be given weights, to comprehensively judge the atomized drug delivery condition and the respiratory condition of the patient through the vital capacity parameter.

With reference to FIG. 6, FIG. 6 is yet another optional flow chart of the control method of the atomized drug delivery system according to an embodiment of the present application, and in some optional embodiments, the system further comprises the upper computer, and the upper computer is connected with the ventilator, the atomizer and the spirometer. The method in FIG. 6 comprises, but is not limited to step S5001 to step S5002.

In step S5001, the test instruction is sent to the ventilator, the atomizer or the spirometer through the test module of the upper computer, so that the ventilator, the atomizer and/or the spirometer is capable of generating the reply information according to the test instruction.

In step S5002, the reply information is sent to the upper computer, the reply information is processed, and the display information and graph obtained by processing are displayed through the display module of the upper computer.

In some embodiments, the control method of the atomized drug delivery system may be executed in the atomized drug delivery system, which means that the upper computer in the control method of the atomized drug delivery system and the atomized drug delivery system of the embodiment shown in FIG 1 both belong to the same inventive concept, so that these embodiments have the same realization principle and technical effect, which will not be described in detail herein.

The embodiments of the present application are provided for the purpose of describing the technical solutions of the present application more clearly, and do not constitute the limitation to the technical solutions of the present application. Those of ordinary skills in the art know that, as the technical evolution and the emergence of new application scenarios, the technical solutions of the present application are also applicable to similar technical problems.

Those of ordinary skills in the art can understand that the technical solutions shown in the drawings does not constitute the limitation to the embodiments of the present application, and may comprise more or less steps than those shown in the drawings, or combine some steps, or comprise different steps.

The system embodiments described above are only illustrative, wherein the units described as separate components may or may not be physically separated, which means that the units may be located in one place or distributed to multiple network units. Some or all of the modules may be selected according to actual needs to achieve the objects of the solutions in the embodiments.

Those of ordinary skills in the art can understand that all or some of the steps in the method, and the functional modules/units in the system, the device disclosed above may be implemented as software, firmware, hardware and an appropriate combination thereof.

The terms "first", "second", "third", "fourth", and the like (if any) in the specification and the drawings of the present application above are used to distinguish similar objects, and are not necessarily used to describe a specific order or sequence. It should be understood that data used in this way may be interchanged under appropriate circumstances, so that the embodiments of the present application described herein can be implemented in a sequence other than those illustrated or described herein. In addition, the terms "comprising", "having" and any variations thereof are intended to cover non-exclusive inclusion. For example, a process, method, system, product or device comprising a series of steps or units is not necessarily limited to those steps or units clearly listed, but may comprise other steps or units not clearly listed in or inherent to the process, method, product or device.

It should be understood that, in the present application, "at least one (item)" and "several" refer to being one or more, and "multiple" refers to being two or more. "And/or" is used for describing the relationship between related objects, and indicates that there may be three relationships. For example, "A and/or B" may indicate that: A exists alone, B exists alone, and A and B both exist, wherein A and B may be singular or plural. The symbol "/" generally indicates that there is a relationship of "or" between the related objects. "At least one (item) of the followings" or similar expression thereof refers to any combination of these items, comprising a singular (item) or any combination of plural (items). For example, at least one (item) of a, b or c may indicate: a, b, c, "a and b", "a and c", "b and c", or "a and b and c", wherein a, b and c may be singular or plural.

In the several embodiments provided in the present application, it should be understood that the disclosed system and method may be implemented in other ways. For example, the system embodiments described above are only illustrative. For example, the division of the units above is only a logical function division. In practice, there may be other division methods. For another example, multiple units or components may be combined or integrated into another system, or some features may be ignored or not executed. In addition, the illustrated or discussed mutual coupling or direct coupling or communication connection may be indirect coupling or communication connection through some interfaces, apparatuses or units, and may be in electrical, mechanical or other forms.

The preferred embodiments of the present application are described above with reference to the drawings, and are not intended to limit the scope of the present application. Any modification, equivalent substitution and improvement made by those of ordinary skills in the art without departing from the scope and essence of the present application should be included within the scope of the present application.

## Claims

1. An atomized drug delivery system, comprising:
a face mask (100), wherein the face mask (100) is connected with a first ventilation pipeline (101) and a second ventilation pipeline (102) respectively, the first ventilation pipeline (101) is connected with a ventilator (110), a respiratory sensor (120) is arranged in the first ventilation pipeline (101), and the respiratory sensor (120) is configured for detecting a respiratory signal;
an atomizer (130), wherein the atomizer (130) is provided with a host machine (131) and an atomizing apparatus (132), the host machine (131) is connected with the atomizing apparatus (132) and the respiratory sensor (120), the host machine (131) is configured for receiving the respiratory signal detected by the respiratory sensor (120), and the atomizing apparatus (132) is further connected with the first ventilation pipeline (101); wherein, the host machine (131) is configured for determining a respiratory state through the respiratory signal, when the respiratory state is an inspiratory state, the host machine (131) is configured for adjusting a frequency of the atomizing apparatus (132), to control the atomizing apparatus (132) to atomize a stored drug and deliver the atomized drug to the first ventilation pipeline (101), and when the respiratory state is an expiratory state, the host machine (131) is configured for adjusting the frequency of the atomizing apparatus (132), to control the atomizing apparatus (132) to stop atomizing the drug;
**characterized in that**,
the atomized drug delivery system comprises a spirometer (140) that is connected with the second ventilation pipeline (102), wherein the spirometer (140) is configured for acquiring exhaled gas through the second ventilation pipeline (102) and calculating a vital capacity parameter according to the gas, to feed back an atomized drug delivery effect;
wherein the host machine is configured to adjust the frequency of the atomizing apparatus to control the atomizing apparatus to reduce a drug atomization amount when a signal magnitude of the respiratory signal is greater than a set respiratory threshold for the system; and
wherein the host machine is configured to adjust the frequency of the atomizing apparatus to control the atomizing apparatus to increase a drug atomization amount when a signal magnitude of the respiratory signal is smaller than the set respiratory threshold for the system.

2. The atomized drug delivery system according to claim 1, further comprising a three-way pipe (150), wherein the three-way pipe (150) is provided with a first interface, a second interface and a third interface, the first interface is connected with the first ventilation pipeline (101), the second interface is connected with the second ventilation pipeline (102), and the third interface is connected with the face mask (100).

3. The atomized drug delivery system according to claim 1, further comprising a filter (160), wherein one end of the filter (160) is connected with the ventilator (110), the other end of the filter (160) is connected with the first ventilation pipeline (101), and the filter (160) is configured for filtering bacteria and impurities.

4. The atomized drug delivery system according to claim 3, further comprising a humidifier (170), wherein one end of the humidifier (170) is connected with the filter (160), the other end of the humidifier (170) is connected with the first ventilation pipeline (101), the humidifier (170) comprises a humidity assembly and a heating assembly, the humidity assembly is configured for adjusting humidity of exhaled gas of the ventilator (110), and the heating assembly is configured for adjusting temperature of the gas.

5. The atomized drug delivery system according to claim 1, further comprising an upper computer (180), wherein the upper computer (180) is connected with the ventilator (110), the atomizer (130) and the spirometer (140), the upper computer (180) comprises a test module and a display module, the test module is configured for sending a test instruction to the ventilator (110), the atomizer (130) and/or the spirometer (140), so that the ventilator (110), the atomizer (130) and/or the spirometer (140) is capable of generating reply information according to the test instruction, and the display module is configured for displaying display information and graph obtained by processing the reply information.

## Patentansprüche

1. Abgabesystem für zerstäubtes Arzneimittel, das Folgendes umfasst:
eine Atemmaske (100), wobei die Atemmaske (100) mit einer ersten Beatmungsleitung (101) bzw. einer zweiten Beatmungsleitung (102) verbunden ist, die erste Beatmungsleitung (101) mit einem Beatmungsgerät (110) verbunden ist, ein Atmungssensor (120) in der ersten Beatmungsleitung (101) angeordnet ist und der Atmungssensor (120) zum Detektieren eines Atemsignals konfiguriert ist;
einen Zerstäuber (130), wobei der Zerstäuber (130) mit einem Host-Rechner (131) und einer Zerstäubungsvorrichtung (132) bereitgestellt ist, der Host-Rechner (131) mit der Zerstäubungsvorrichtung (132) und dem Atmungssensor (120) verbunden ist, der Host-Rechner (131) zum Empfang des Atemsignals, das durch den Atmungssensor (120) detektiert wird, konfiguriert ist und die Zerstäubungsvorrichtung (132) weiter mit der ersten Beatmungsleitung (101) verbunden ist; wobei der Host-Rechner (131) zum Bestimmen eines Atemzustands durch das Atemsignal konfiguriert ist, wenn der Atemzustand ein inspiratorischer Zustand ist, der Host-Rechner (131) zum Anpassen einer Frequenz der Zerstäubungsvorrichtung (132) konfiguriert ist, um die Zerstäubungsvorrichtung (132) zu steuern, um ein eingelagertes Arzneimittel zu zerstäuben und das zerstäubte Arzneimittel an die erste Beatmungsleitung (101) abzugeben, und wenn der Atemzustand ein exspiratorischer Zustand ist, der Host-Rechner (131) zum Anpassen der Frequenz der Zerstäubungsvorrichtung (132) konfiguriert ist, um die Zerstäubungsvorrichtung (132) zu steuern, um das Zerstäuben des Arzneimittels zu stoppen;
**dadurch gekennzeichnet, dass**
das Abgabesystem für zerstäubtes Arzneimittel ein Spirometer (140) umfasst, das mit der zweiten Beatmungsleitung (102) verbunden ist, wobei das Spirometer (140) zur Erfassung von ausgeatmetem Gas durch die zweite Beatmungsleitung (102) und Berechnung eines Vitalkapazitätsparameters entsprechend dem Gas konfiguriert ist, um einen Abgabeeffekt von zerstäubtem Arzneimittel zurückzumelden;
wobei der Host-Rechner zum Anpassen der Frequenz der Zerstäubungsvorrichtung konfiguriert ist, um die Zerstäubungsvorrichtung zu steuern, um eine Arzneimittelzerstäubungsmenge zu reduzieren, wenn eine Signalgröße des Atemsignals größer ist als eine festgesetzte Atemschwelle für das System; und
wobei der Host-Rechner zum Anpassen der Frequenz der Zerstäubungsvorrichtung konfiguriert ist, um die Zerstäubungsvorrichtung zu steuern, um eine Arzneimittelzerstäubungsmenge zu erhöhen, wenn eine Signalgröße des Atemsignals kleiner ist als die festgesetzte Atemschwelle für das System.

2. Abgabesystem für zerstäubtes Arzneimittel nach Anspruch 1, das weiter eine Dreiwege-Leitung (150) umfasst, wobei die Dreiwege-Leitung (150) mit einer ersten Anschlussstelle, einer zweiten Anschlussstelle und einer dritten Anschlussstelle bereitgestellt ist, die erste Anschlussstelle mit der ersten Beatmungsleitung (101) verbunden ist, die zweite Anschlussstelle mit der zweiten Beatmungsleitung (102) verbunden ist und die dritte Anschlussstelle mit der Atemmaske (100) verbunden ist.

3. Abgabesystem für zerstäubtes Arzneimittel nach Anspruch 1, das weiter einen Filter (160) umfasst, wobei ein Ende des Filters (160) mit dem Beatmungsgerät (110) verbunden ist, das andere Ende des Filters (160) mit der ersten Beatmungsleitung (101) verbunden ist und der Filter (160) zum Filtern von Bakterien und Verunreinigungen konfiguriert ist.

4. Abgabesystem für zerstäubtes Arzneimittel nach Anspruch 3, das weiter einen Luftbefeuchter (170) umfasst, wobei ein Ende des Luftbefeuchters (170) mit dem Filter (160) verbunden ist, das andere Ende des Luftbefeuchters (170) mit der ersten Beatmungsleitung (101) verbunden ist, der Luftbefeuchter (170) eine Luftbefeuchtungseinheit und eine Erwärmungseinheit umfasst, die Luftbefeuchtungseinheit zum Anpassen der Luftfeuchtigkeit von ausgestoßenem Gas des Beatmungsgeräts (110) konfiguriert ist und die Erwärmungseinheit zum Anpassen der Temperatur des Gases konfiguriert ist.

5. Abgabesystem für zerstäubtes Arzneimittel nach Anspruch 1, das weiter einen übergeordneten Computer (180) umfasst, wobei der übergeordnete Computer (180) mit dem Beatmungsgerät (110), dem Zerstäuber (130) und dem Spirometer (140) verbunden ist, der übergeordnete Computer (180) ein Prüfmodul und ein Anzeigemodul umfasst, das Prüfmodul zum Senden einer Prüfanweisung an das Beatmungsgerät (110), den Zerstäuber (130) und/oder das Spirometer (140) konfiguriert ist, sodass das Beatmungsgerät (110), der Zerstäuber (130) und/oder das Spirometer (140) Antwortinformationen entsprechend der Prüfanweisung erzeugen können, und das Anzeigemodul zum Anzeigen von Anzeigeinformationen und grafischer Darstellung, die durch Verarbeiten der Antwortinformationen erhalten werden, konfiguriert ist.

## Revendications

1. Système d'administration de médicament atomisé, comprenant :
un masque facial (100), le masque facial (100) étant relié à un premier conduit de ventilation (101) et un second conduit de ventilation (102) respectivement, le premier conduit de ventilation (101) étant relié à un ventilateur (110), un capteur de respiration (120) étant disposé dans le premier conduit de ventilation (101), et le capteur de respiration (120) étant conçu pour détecter un signal respiratoire ;
un atomiseur (130), l'atomiseur (130) étant pourvu d'une machine hôte (131) et d'un appareil d'atomisation (132), la machine hôte (131) étant reliée à l'appareil d'atomisation (132) et au capteur de respiration (120), la machine hôte (131) étant conçue pour recevoir le signal respiratoire détecté par le capteur de respiration (120), et l'appareil d'atomisation (132) étant en outre relié au premier conduit de ventilation (101) ; la machine hôte (131) étant conçue pour déterminer un état respiratoire à travers le signal respiratoire, quand l'état respiratoire est un état inspiratoire, la machine hôte (131) est conçue pour ajuster une fréquence de l'appareil d'atomisation (132), pour commander l'appareil d'atomisation (132) afin d'atomiser un médicament stocké et fournir le médicament atomisé au premier conduit de ventilation (101), et quand l'état respiratoire est un état expiratoire, la machine hôte (131) est conçue pour ajuster la fréquence de l'appareil d'atomisation (132), pour commander l'appareil d'atomisation (132) afin de stopper l'atomisation du médicament ;
**caractérisé en ce que**,
le système d'administration de médicament atomisé comprend un spiromètre (140) qui est relié au second conduit de ventilation (102), le spiromètre (140) étant conçu pour acquérir un gaz exhalé à travers le second conduit de ventilation (102) et calculer un paramètre de capacité vitale en fonction du gaz, pour alimenter en retour un effet d'administration de médicament atomisé ;
la machine hôte étant conçue pour ajuster la fréquence de l'appareil d'atomisation afin de commander l'appareil d'atomisation pour réduire une quantité d'atomisation de médicament quand l'amplitude de signal du signal respiratoire est supérieure à un seuil respiratoire défini pour le système ; et
la machine hôte étant conçue pour ajuster la fréquence de l'appareil d'atomisation afin de commander l'appareil d'atomisation pour augmenter une quantité d'atomisation de médicament quand l'amplitude de signal du signal respiratoire est inférieure au seuil respiratoire défini pour le système.

2. Système d'administration de médicament atomisé selon la revendication 1, comprenant en outre un tuyau à trois voies (150), le tuyau à trois voies (150) étant pourvu d'une première interface, d'une deuxième interface et d'une troisième interface, la première interface étant reliée au premier conduit de ventilation (101), la deuxième interface étant reliée au second conduit de ventilation (102), et la troisième interface étant reliée au masque facial (100).

3. Système d'administration de médicament atomisé selon la revendication 1, comprenant en outre un filtre (160), une extrémité du filtre (160) étant reliée au ventilateur (110), l'autre extrémité du filtre (160) étant relié au premier conduit de ventilation (101), et le filtre (160) étant conçu pour filtre les bactéries et les impuretés.

4. Système d'administration de médicament atomisé selon la revendication 3, comprenant en outre un humidificateur (170), une extrémité de l'humidificateur (170) étant reliée au filtre (160), l'autre extrémité de l'humidificateur (170) étant reliée au premier conduit de ventilation (101), l'humidificateur (170) comprenant un ensemble d'humidité et un ensemble de chauffage, l'ensemble d'humidité étant conçu pour ajuster l'humidité du gaz exhalé du ventilateur (110), et l'ensemble de chauffage étant conçu pour ajuster la température du gaz.

5. Système d'administration de médicament atomisé selon la revendication 1, comprenant en outre un ordinateur pilote (180), l'ordinateur pilote (180) étant relié au ventilateur (110), à l'atomiseur (130) et au spiromètre (140), l'ordinateur pilote (180) comprenant un module de test et un module d'affichage, le module de test étant conçu pour envoyer une instruction de test au ventilateur (110), à l'atomiseur (130) et/ou au spiromètre (140), de telle sorte que le ventilateur (110), l'atomiseur (130) et/ou le spiromètre (140) puissent générer des informations de réponse en fonction de l'instruction de test, et le module d'affichage est conçu pour afficher des informations et un graphique d'affichage obtenus par un traitement des informations de réponse.
